# NEUE EUROPÄISCHE PATENTSCHRIFT

(11) **EP 0 946 818 B2**
(45) Veröffentlichungstag und Bekanntmachung des Hinweises auf die Entscheidung über den Einspruch: **16.09.2009**
(45) Hinweis auf die Patenterteilung: 20.03.2002
(21) Anmeldenummer: 97953657.0
(22) Anmeldetag: 19.12.1997
(51) Int. Cl.: D21C 9/10

(54) **VERFAHREN UND VORRICHTUNG ZUR PROZESSFÜHRUNG BEIM BLEICHEN VON FASERSTOFFEN**
METHOD AND DEVICE FOR PROCESS CONTROL DURING BLEACHING OF FIBROUS MATERIALS
PROCEDE ET DISPOSITIF POUR CONDUIRE UN PROCESSUS LORS DU BLANCHIMENT DE MATIERES FIBREUSES

(30) Priorität: 20.12.1996 DE 19653479
(43) Veröffentlichungstag der Anmeldung: 06.10.1999
(73) Patentinhaber: SIEMENS AKTIENGESELLSCHAFT, 80333 München (DE)
(72) Erfinder: FURUMOTO, Herbert, D-91052 Erlangen (DE); OBRADOVIC, Dragan, D-81669 München (DE)
(86) Internationale Anmeldenummer: PCT/DE1997/002988
(87) Internationale Veröffentlichungsnummer: WO 1998/028488

(56) Entgegenhaltungen:
- WO-A-95/08019
- WO-A-96/12183
- DE-A- 19 510 008
- SE-A- 9 403 520
- US-A- 3 980 517
- US-A- 4 013 506
- 'Pulp characterization', 1991, UNIVERSITY OF UMEÅ, UMEÅ, ISBN 91-7174-578-5 Artikel LARS WALLBÄCKS: 'using spectroscopy and multivate data analysis'

## Beschreibung

Die Erfindung bezieht sich auf ein Verfahren zur Prozeßführung und zur Prozeßoptimierung beim Bleichen von Faserstoffen, insbesondere Zellstoff, Holzstoff oder Altpapierstoff, durch Behandeln einer Suspension des Faserstoffes mit Bleichchemikalien unter Einsatz eines Zustandsmodells und/oder Prozeßmodells. Daneben bezieht sich die Erfindung auf eine Vorrichtung zur Durchführung dieses Verfahrens.

Bei der Herstellung von Papier stellt das Bleichen von Faserstoffen einen wichtigen Verfahrensschritt dar. Unter Faserstoffen wird dabei der durch Kochen von Holzschnitzeln in Kochflüssigkeit hergestellte Zellstoff, der mechanisch erzeugte Holzstoff - wie CMT-Stoff (chemical, mechanical, thermomechanical) oder Refiner-Stoff bzw. Holzschliff - und/oder Altpapierstoff verstanden, die nachfolgend pauschal als "Stoff" bezeichnet werden. Beim Bleichen geht es um die Erhöhung des Weißegrades der Stoffe, die aber eine ausreichende Festigkeit behalten sollen. Dabei werden gleichermaßen lösliche Ligninverbindungen gebildet und die Delignifizierung fortgesetzt. Weiterhin sollen färbende Substanzen zerstört werden. Scmit ist der Bleichprozeß eine Fortsetzung des Holzaufschlusses.

Zur Gewährleistung des geforderten Wirkungsgrades sind Bleichvorrichtungen mit mehreren Stufen ausgelegt. In der Praxis existieren üblicherweise wenigstens zweistufige Bleichvorrichtungen, wobei die Anzahl der Stufen nicht begrenzt ist. Dabei kommt es darauf an, daß bei der Prozeßführung die Prozeßvariablen aufeinander abgestimmt und hinsichtlich des Wirkungsgrades und/oder Ausbeute optimiert werden.

In der WO 96/12183 A1 wird eine Methode zur Bestimmung der organischen Bestandteile in Stoffsuspensionen, die bei der Zellstoff- und Papiererzeugung anfallen, beschrieben. Dabei werden spektroskopische Methoden, insbesondere im Bereich UV/VIS/NIR/IR einschließlich Raman-Spektroskopie angewandt und durch Auswertung und Datenanalyse der erhaltenen Spektren mittels chemometrischer Methoden interessierende Substanzen und deren Eigenschaften ermittelt. Weiterhin wird in der US 4 013 506 A eine Methode und ein zugehöriges Verfahren zur Regelung der Viskosität und Helligkeit einer Zellstoffpulpe während eines mehrstufigen Bleichvorganges beschrieben. Dabei sollen optische Methoden zum Einsatz kommen: Insbesondere wird bei Einstrahlung von Licht im vorgegebenen Wellenlängenbereich das reflektierte Licht erfaßt und mittels klassischer Methoden die Summen, Differenzen oder Anteilverhältnisse von reflektiertem Licht unterschiedlicher Wellenlängenbandbreiten bestimmt. Daraus werden Aussagen über den Grad des Bleichens abgeleitet. Schließlich soll gemäß der US 3 980 517 A in einer mehrstufigen Anlage zum Bleichen die Wirksamkeit der Bleichchemikalien durch optische Methoden bestimmt werden. Dabei wird Strahlung einer Wellenlänge eingestrahlt und die Streuung des reflektierten Lichtes bestimmt, wodurch Aussagen über den Zustand der Bleichchemikalien gemacht werden können.

Aufgabe der Erfindung ist es, die Prozeßführung beim Bleichvorgang zu optimieren und eine zugehörige Vorrichtung zu schaffen.

Die Aufgabe ist bei einem Verfahren der eingangs genannten Art erfindungsgemäß mit den Merkmalen des Patentanspruches 1 gelöst, wobei vorteilhafte Weiterbildungen in den abhängigen Ansprüchen angegeben sind. Die zugehörige Vorrichtung ist im einzigen Sachanspruch angegeben.

Bei der Erfindung werden an wenigstens einer Stelle kontinuierliche Spektren von optischen und/oder mechanischen Eigenschaften in der Stoffsuspension oder an einem aus der Stoffsuspension erzeugten Zellstoffblatt gemessen. Die Auswertung dieser Spektren erfolgt beispielsweise mit algebraischen Methoden und/oder mit neuronalen Netzen.

Die Anwendung von spektroskopischen Messungen in der Papierindustrie ist zwar bereits schon vorgeschlagen worden. Beispielsweise wird in der WO 95/08019 A1 ein Zustandsanalysator für eine Altpapiersuspension verwendet, der aus einem Spektrometer und einem neuronalen Netz aufgebaut ist. Dabei werden aus Kennwerten der Altpapiersuspension Steuergrößen für die nachfolgende Altpapieraufbereitung abgeleitet. Weiterhin ist aus der DE 195 10 008 A1 eine Meßeinrichtung zur Erfassung spektraler und/oder physikalischer Kennwerte mit einem nachfolgenden neuronalen Netz bekannt, bei der mit der Meßeinrichtung wenigstens die Ausgangsstoffe der Zellstoff- und Papierherstellung erfaßt werden.

Speziell der Bleichvorgang ist letzteren Stand der Technik nicht angesprochen. Allerdings wird die Aufnahme und Auswertung von optischen Spektren bei Anlagen in der Papierindustrie in den internationalen Anmeldungen WO 93/15 389 A1, WO 94/01 769 A1 und WO 95/31 709 A1 beschrieben. Im einzelnen sind diese Druckschriften auf die Auswertung von Spektren mittels der sogenannten Hauptkomponentenanalyse gerichtet.

Demgegenüber kommt es bei der Erfindung darauf an, speziell an genau definierten Stellen der Bleichanlage die spektroskopischen Messungen vorzunehmen und mit geeigneten, jeweils auf die spezifische Meßsituation abgestellten Methoden auszuwerten. Neben der Erfassung von insbesondere optischen Spektren aus elektromagnetischer Strahlung können auch beispielsweise Spektren der Faserlängenverteilung als mechanische Eigenschaften zur Auswertung herangezogen werden, woraus sich Kenngrößen bzw. Zustandsmaßzahlen ableiten lassen. Insbesondere eine Kombination mit diskreten physikalischen und/oder chemischen Eigenschaften führt dazu, daß sich neben der Weiße auch die Festigkeitseigenschaften modellieren lassen. Daraus lassen sich Steuergrößen für die Prozeßführung- und Optimierung beim Bleichen von Faserstoffen ableiten.

Die Auswertung der Spektren kann vorteilhafterweise mit einer Hauptkomponentenanalyse erfolgen, wobei algebraische Rechenmethoden eingesetzt werden. Auch andere Methoden haben sich als geeignet erwiesen.

Sofern mit elektromagnetischen Wellen im Bereich der Wellenlängen zwischen 100 nm und 400 µm, vorzugsweise im Bereich von 0,4 µm bis 100 µm, gearbeitet wird, können Absorptions-, Emissions-, Lumineszenz- oder Ramanspektren gemessen werden. Die Absorptionsspektroskopie kann in Transmission, diffuser Reflexion oder gedämpfter Totalreflexion (ATR = atennuated total reflection) erfolgen. Die Anregung zur Lumineszenz kann z.B. durch die Einstrahlung von elektromagnetischer Strahlung erfolgen (z.B. UV-Strahlung) oder durch eine spezifische chemische Reaktion (Chemolumineszenz), die Anregung der Emission dagegen z.B. durch Bestrahlung mit Elektronen erfolgen. Bei Messung im Bereich des Infraroten (800 nm bis 20 µm) kann vorzugsweise die Fourier-Transformations-Infrarot-Spektroskopie (FTIR) eingesetzt werden. Bei inhomogenen Proben erfolgt zur Hermsistenz die spektroskopische Messung mehrfach.

Vorzugsweise werden die Spektren wie folgt vorverarbeitet:
- durch Fourier-Transformation.
- Bei der Messung der Absorption durch diffuse Reflexion durch Umrechnung in Kubelka-Munk-Einheiten und Korrektur von Mehrfachstreueffekten
- durch Normierung und Glättung der Spektren
- durch Ermittlung von für die Modellbildung ungeeigneten Spektren. Die Ausschaltung ungeeigneter Messungen kann z.B. durch Vergleich mit Referenzspektren erfolgen
- durch Bildung von Mittelwerten bei mehreren Spektren zu einer Probennahme.

Nach diesen ersten Verarbeitungsschritten werden aus Spektren ganz oder abschnittsweise folgende rechnergestütze Verfahren zur Ermittlung von Kenngrößen angewandt, wobei die Beschreibung der Spektren im wesentlichen durch ihre Hauptkomponenten erfolgt,
- Hauptkomponentenanalyse (PCA)
- Partial Least Square (PLS)-Verfahren
- Neuronale Netze
- Analytische Beschreibung der Spektren, z.B. im Bereich des IR durch Lage, Intensität und Breite der wichtigsten Absorptions- oder Emissionspeaks, Ermittlung dieser Größen z.B. mit einfachen Minimum-/Maximum-Verfahren oder der 2. Ableitung.
Die Kenngrößen werden zur Modellierung der gewünschten Qualitätsparameter herangezogen.

Die Zustandsmodelle zur Berechnung der Qualitätsparameter können bei ausreichend großer Zahl von Daten auf der Basis von neuronalen Netzen, Fuzzy-Systemen, Multilinearen Regressionsmodellen bzw. Kombinationen daraus strukturiert sein. Alternativ zu rein datengetriebenen Modellen sind auch kombinierte Modelle möglich, bei denen zusätzlich analytisches Wissen eingebracht wird. Auf die gleiche Weise und mit den gleichen Mitteln können die diesbezüglichen Prozeßmodelle aufgebaut werden.

Die Aufstellung der Modelle erfolgt mit Labormessungen am Zwischen- und Endprodukt. Das Training der Modelle erfolgt jeweils auf der Grundlage der Laborwerte und kann in bestimmten Zeitabständen wiederholt werden, wobei auch ein nur partielles Nachlernen möglich ist. Weiterhin kann eine in der Spektrenvorverarbeitung integrierte Prüfung auf Modellgültigkeit ("Novelty Detection") entsprechend der älteren, nicht vorveröffentlichten DE 196 322 45 A1 im laufenden Prozeß rechtzeitig die Notwendigkeit einer neuen Trainingsphase anzeigen.

Weitere Einzelheiten und Vorteile der Erfindung ergeben sich aus der nachfolgenden Figurenbeschreibung von Ausführungsbeispielen in Verbindung mit den weiteren Unteransprüchen. Es zeigen
- Figur 1: ein Schema der Meßstellen bei einer zweistufigen Bleiche,
- Figur 2a und 2b: Spektren von optischen Messungen an einer Faserstoffsuspension und von mechanischen Messungen an Fasern,
- Figur 3: ein Zustandsmodell der Qualitätsparameter,
- Figur 4: ein Prozeßmodell der Endweiße in Figur 1,
- Figur 5: ein Prozeßmodell der sogenannten Schmutzpunkte in Figur 1,
- Figur 6: ein dynamisches Modell für die Weiße,
- Figur 7: den schematischen Aufbau einer Prozeßoptimierung zum Steuern des Bleichvorganges,
- Figur 8: eine Alternative des Prozeßmodells gemäß zu Figur 4,
- Figur 9: eine Anordnung zur Verarbeitung der Spektren aus Figur 1,
- Figur 10: ein mehrstufiges statisches neuronales Modell und
- Figur 11: die komplette Vorrichtung zur optimierten Prozeßführung des Bleichvorganges.

Die Figuren werden nachfolgend teilweise gemeinsam beschrieben. Gleiche bzw. gleichwirkende Teile haben sich entsprechende Bezugszeichen.

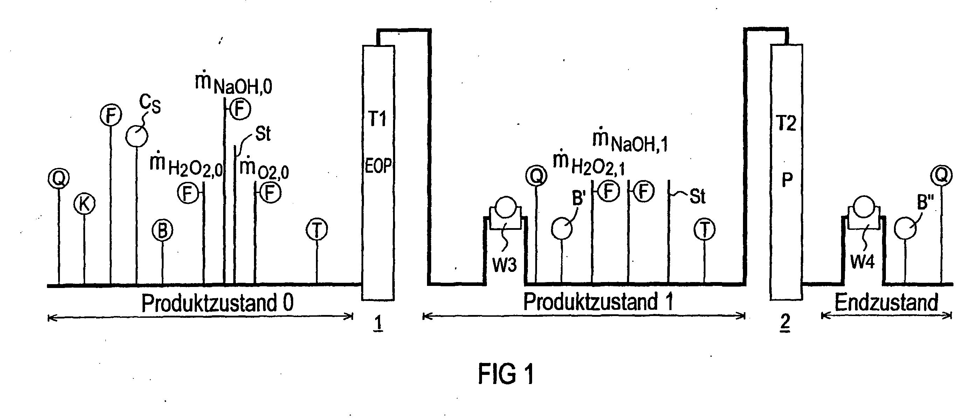

In Figur 1 ist die Instrumentierung speziell einer zweistufigen Bleiche verdeutlicht, da in der Regel derartige Bleichen aus mehreren Bleichstufen bestehen: Eine erste Bleichstufe ist mit EOP und eine zweite Bleichstufe ist mit P bezeichnet, wobei der Ausgangsstoff aus einem Produktzustand 0 über einen Prozeß 1 in den Produktzustand 1 und über einen Prozeß 2 in den Endzustand gebracht wird. Prozeßmeßgrößen im Zustand 0 sind üblicherweise neben der Weiße B die sogenannte Kappazahl K, der Durchfluß F des Materials, die Konsistenz Cs als sog. Feststoffkonzentration, der Massefluß *m*_{*H*₂*O*₂},0, der Massefluß *m_{NaOH,}*0, der Dampfstrom St, der Massestrom *m*_{*o*₂},0 und die Temperatur T, wobei nunmehr an der Meßstelle Q die kontinuierlichen Spektren elektromagnetischer und/oder mechanischer Größen gemessen werden.

Im Produktzustand 1 wird bei der ersten Bleichsequenz W3 als Prozeßmeßgröße der bereits erreichte Weißegrad B sowie die Masseströme *mᵢ*, 1 von H₂O₂ und NaOH sowie der Dampf St und die Temperatur erfaßt. Auch an dieser Stelle können gegebenenfalls wiederum kontinuierliche Spektren optischer und/oder mechanischer Art erfaßt werden. Gleiches gilt in der zweiten Bleichsequenz W4 nach dem Prozeß 2 für die Endweiße B im Endzustand des gebleichten Faserstoffes, wobei wiederum ein Spektrum Q gemessen wird.

In der Zellstoffbleiche werden neben der Weiße weitere Qualitätswerte des Zellstoffes spektrometrisch erfaßt. Die Kennwerte des Spektrums, wie z.B. die Hauptkomponenten des Spektrums, werden anschließend ausgewertet und finden neben weiteren Prozeßgrößen Eingang in ein Modell für die Qualität des in der Bleicherei bearbeiteten Zellstoffes.

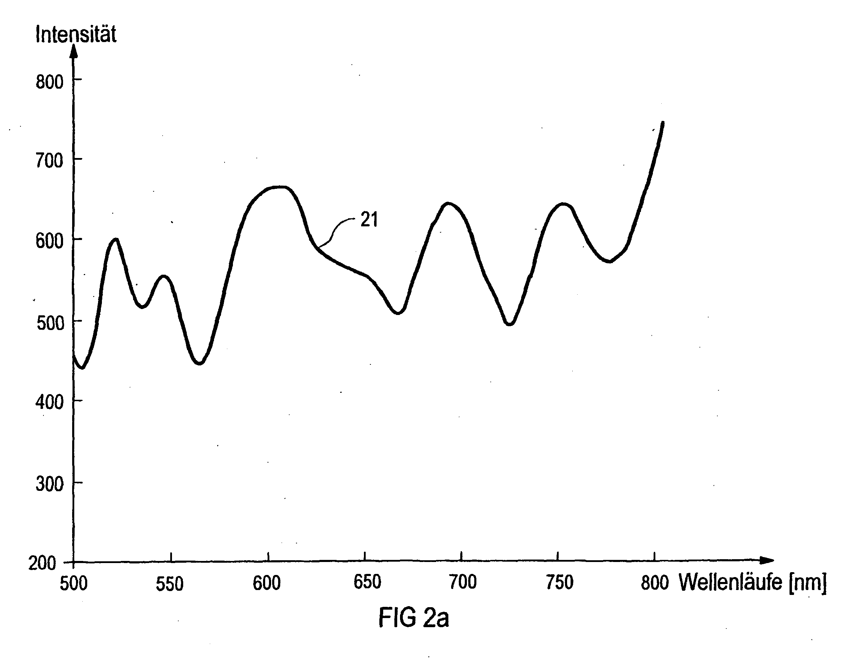

Ein derartiges optisches Spektrum 21 ist beispielhaft als Infrarot-Spektrum in Figur 2a dargestellt. Prinzipiell sind alle Spektren elektromagnetischer Strahlung mit Wellenlängen zwischen 100 nm und 400 µm möglich, wobei bei den Spektren als Abszisse die Wellenzahl cm⁻¹ dargestellt ist. Die elektromagnetische Strahlung können als Absorptions-, Emissions-, Lumineszenz- oder als Raman-Spektren gemessen werden. Die Erfassung der Strahlung ist in Transmission, direkter oder diffuser Reflexion, aber auch in gedämpfter Totalreflexion (ATR) möglich.

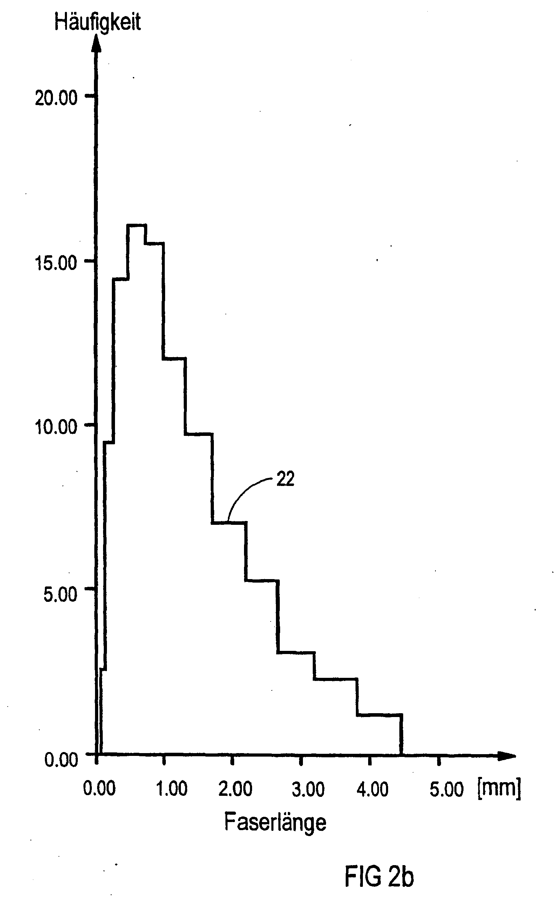

Alternativ zu optischen Messungen, die bei signifikanten Wellenlängen jeweils bestimmte Peaks aufweisen, können auch Spektren von mechanischen Eigenschaften, wie beispielsweise der Faserlängenverteilung der Fasern, erfaßt werden. Hier ergibt sich in etwa ein Spektrum 22 gemäß Figur 2b, bei dem die Begrenzung der Balkendarstellung in etwa eine Bernoulli-Verteilung wiedergibt. Die Verteilung der Siebfraktionen einzelner Fasern des Faserstoffes hat einen ähnlichen Verlauf.

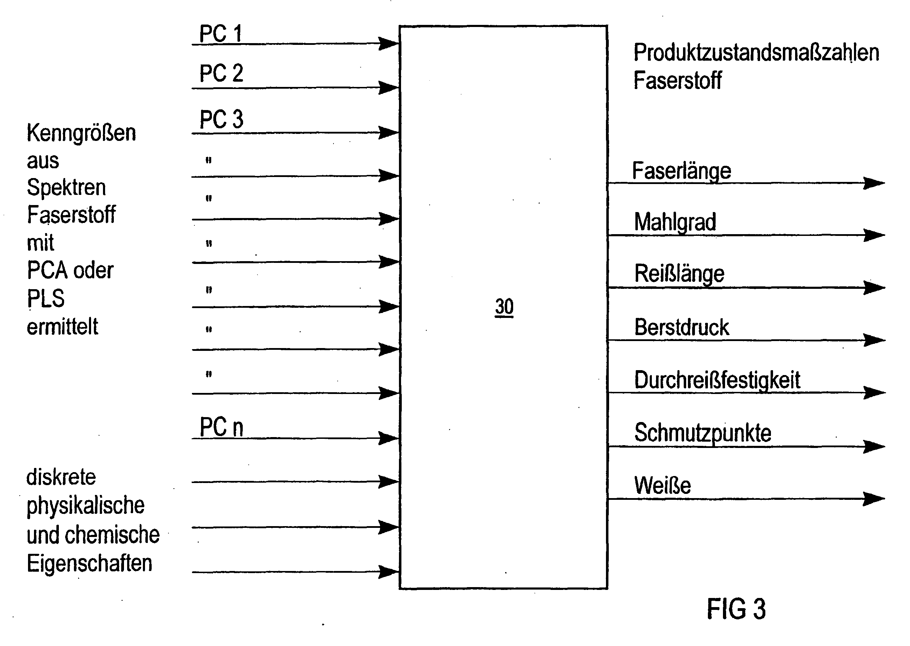

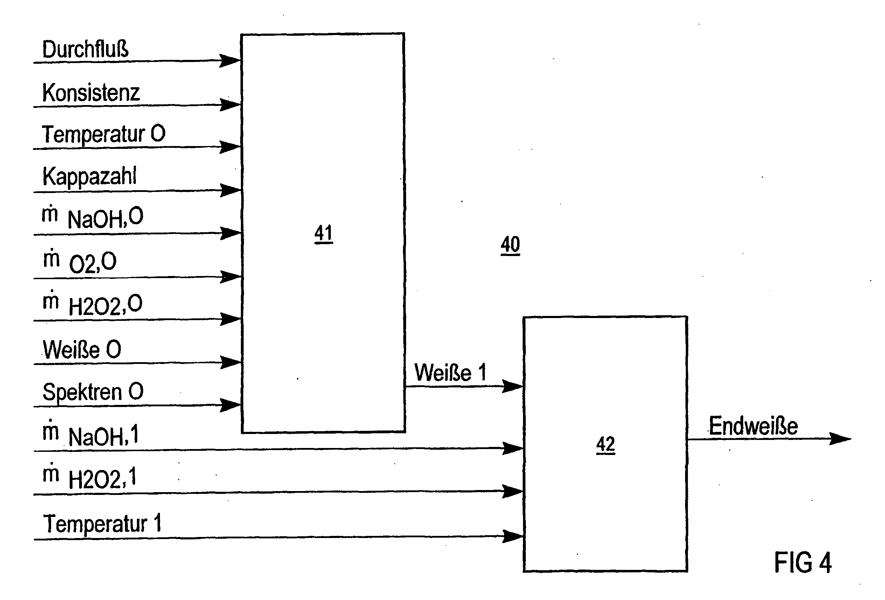

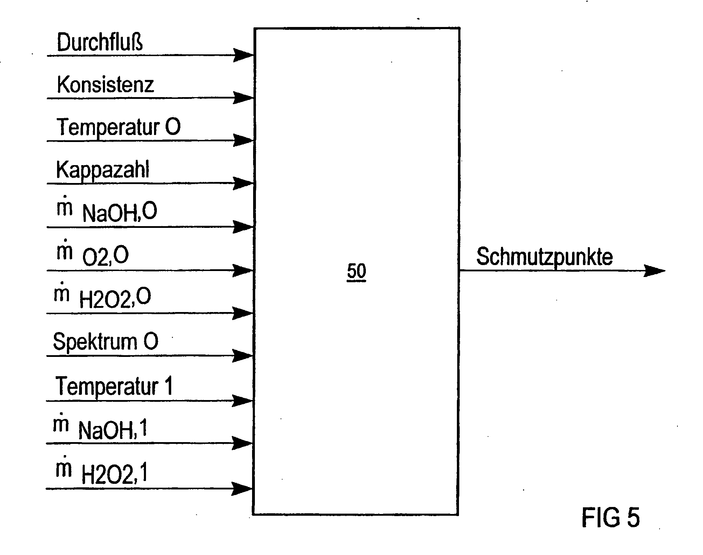

In Figur 3 ist ein Zustandsmodell 30 für die Qualität des erzeugten Zellstoffes dargestellt, das mit den Prozeßeigenschaften zu einem Prozeßmodell ergänzt werden kann. Das Zustandsmodell 30 kann ebenso wie die Bleiche in mehreren Stufen, beispielsweise zwei Stufen, unterteilt sein, was in Figur 4 als Prozeßmodell 40 beispielhaft für die Endweiße dargestellt ist. Derartige Modelle können aber auch einstufig aufgebaut sein, wie es in Figur 5 für die Schmutzpunkte dargestellt ist. Für die Bildung der Zustands- und/oder Prozeßmodelle werden ggfs auch diskrete physikalische und /oder chemische Eigenschaften des Faserstoffes, der Stoffsuspension bzw.des Probenblattes herangezogen.

Modelliert werden können neben der Weiße und den Schmutzpunkten auch die Festigkeitswerte des Zellstoffes, wie die Reißlänge oder der sogenannte Berstdruck. Zur Modellierung werden im Prinzip klassische Modellierungsmethoden verwendet. Insbesondere können aber neuronale Netze eingesetzt werden.

Neben der Vorhersage und Simulation der Qualitätseigenschaften werden die Modelle zur Optimierung der Stellgrößen, wie zur Bestimmung der Temperaturen in den einzelnen Bleichtürmen und für die Dotierung der Bleichchemikalien, herangezogen. Dabei kann eine Optimierung beispielsweise mit genetischen Algorithmen erfolgen. Selbstverständlich können auch konventionelle Techniken eingesetzt werden.

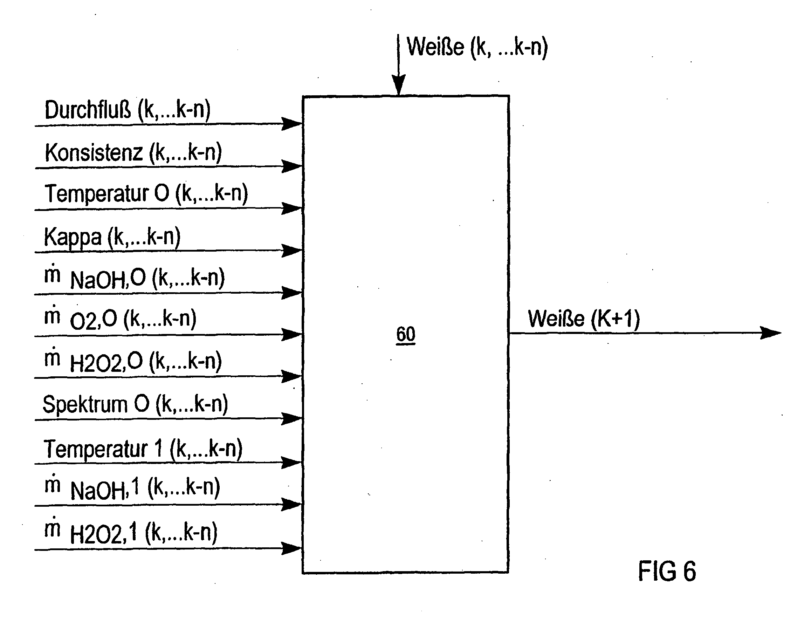

In Figur 6 ist das dynamische Modell der Bleiche dargestellt. Anhand von historischen Daten zu den Zeitpunkten k bis (k-n) wird beispielhaft die Weiße zum Zeitpunkt (k+1) vorhergesagt. Vorteilhaft wird das dynamische Modell mit einem neuronalen Netz erstellt.

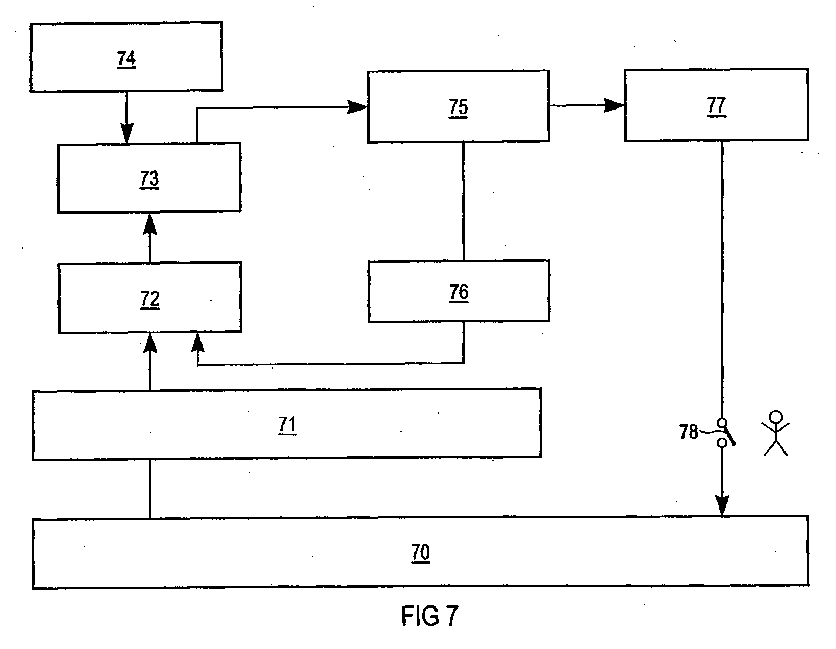

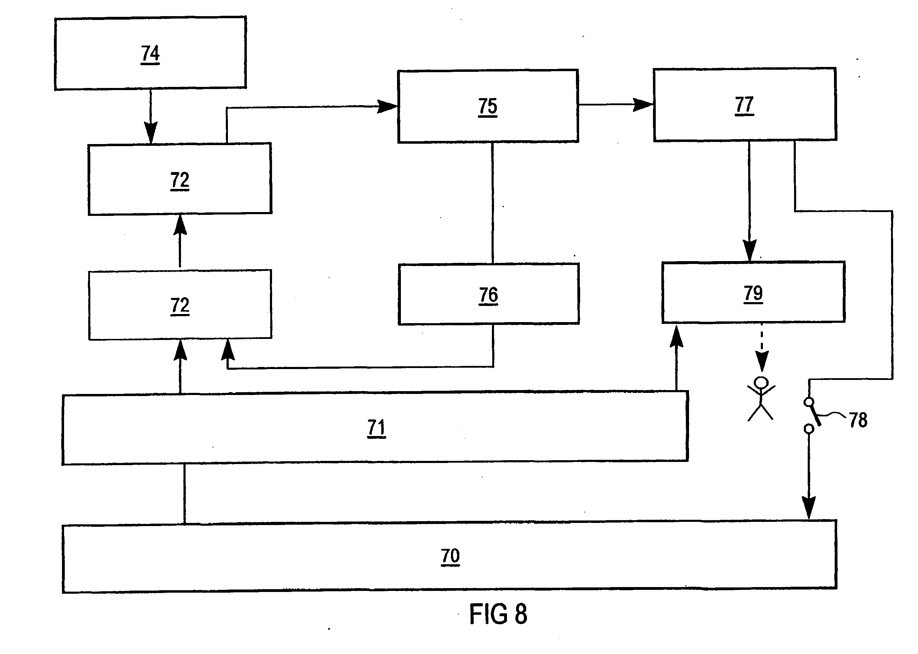

Möglichkeiten der Prozeßoptimierung sind beispielhaft in Figur 7 und 8 zu sehen. Dabei werden vom Prozeß Meßwerte von Prozeßzuständen übernommen, wie Temperaturen, Drücke, Konsistenz, Durchflüsse usw., und neben den zu optimierenden Stellgrößen dem anhand der Figuren 3, 4 und 5 beschriebenen Modell zugeführt. Die in den Figuren 3 bis 5 jeweils dargestellten Modelle 30, 40 und 50 berechnen die Qualitätskennwerte. In einem eigenen weiteren Schritt wird eine Kostenfunktion gebildet, die aus den Chemikalieneinsätzen und den Temperaturen die Produktionskosten berechnen. Gleichzeitig können neben den reinen Produktionskosten die Produktqualitäten preislich bewertet werden. Ein Optimierer, der z.B. nach der Gradientenmethode arbeitet, optimiert durch Variation der Stellgrößen die Kostenfunktion.

Die Optimierung kann dadurch durchgeführt werden, daß zur Ermittlung der optimalen Stellgrößen ein statisches Modell eingesetzt wird und die optimalen Stellgrößen einem dynamischen Modell zugeführt werden, bevor sie zum Prozeß durchgeschaltet werden. Letzteres ist in Figur 8 dargestellt. Das dynamische Modell überprüft die optimalen Stellgrößen bei k, ..., (k-n) und liefert somit den Übergang vom gegenwärtigen Arbeitspunkt zu dem von der Optimierung vorgegebenen Arbeitspunkt (k+1). Der Anlagenfahrer kann entscheiden, ob der neue Arbeitspunkt akzeptiert werden kann.

Die Figuren 7 und 8 zeigen das Ergebnis der Modellierungsstrategien zur Prozeßoptimierung. Der allgemeine Prozeß ist hier mit 70 bezeichnet, woraus sich der aktuelle Prozeßzustand 71 anhand der Spektren ergibt. Das Prozeßmodell ist mit 72 bezeichnet, aus dem die Daten in eine Einheit mit Kostenfunktion 73 gegeben werden, die gleichzeitig mit Daten für Kosten und Preise aus der Einheit 74 beaufschlagt wird.

Ein Optimierer 75 ermittelt daraus die Stellgrößen 76, die in das Prozeßmodell 72 zurückgekoppelt werden und weiterhin die optimalen Stellgrößen 77 zur Prozeßführung. Diese können über einen Schalter 78 vom Anlagenfahrer durchgeschaltet werden, sofern sie als sinnvoll erkannt werden.

In Figur 8 ist Figur 7 insofern ergänzt, daß gleichermaßen ein dynamisches Modell entsprechend Figur 6 verwendet wird. Hier ist zusätzlich eine Einheit 79 mit dem dynamischen Modell vorhanden, in die der aktuelle Prozeßzustand einerseits und die optimalen Stellgrößen 77 andererseits eingegeben werden.

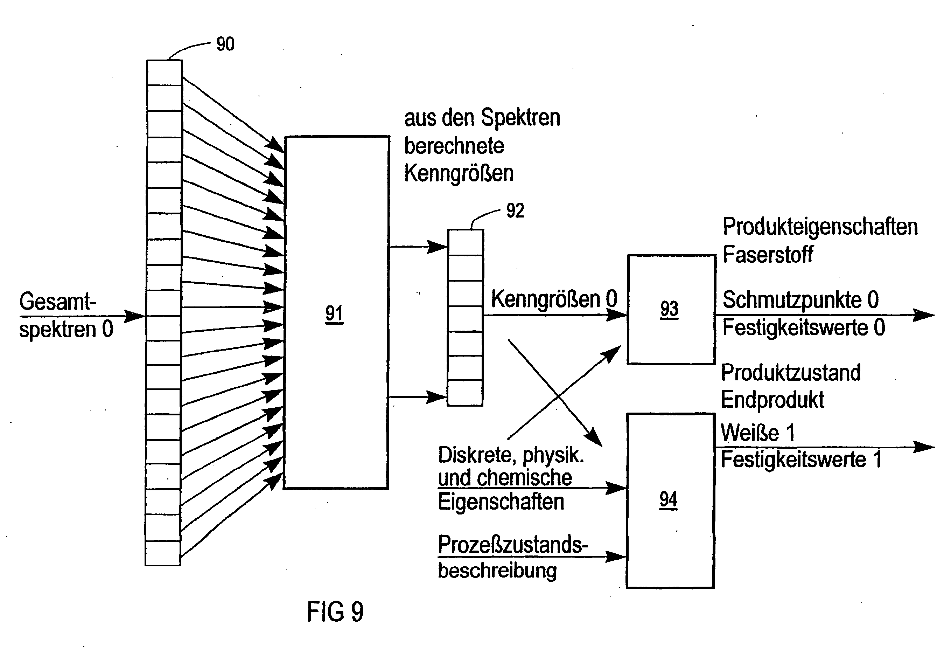

Es ist sinnvoll, die Spektren vorzuverarbeiten und die vorverarbeiteten Signale jeweils bei der nachfolgenden Modellierung zu nutzen. In Figur 9 ist dargestellt, daß eine Einheit 91 zur Vorverarbeitung und Verdichtung des Gesamtspektrums dient, aus dem entsprechend Einheit 92 die Kenngrößen berechnet werden. Die Kenngrößen fließen in das Zustandsmodell 93 und in das Prozeßmodell 94 ein, wobei zusätzlich diskrete mechanische und/oder chemische Eigenschaften eingegeben werden können und die Prozeßzustandsbeschreibung das Zustandsmodells zum Prozeßmodell ergänzen. Vom Zustandsmodell werden die Qualitätsparameter für die Bleiche und aus dem Prozeßmodell der Qualitätsparameter für das Endprodukt abgeleitet.

Die Vorverarbeitungsmethoden erzeugen also ein verdichtetes Spektrum und werden beispielsweise nach Art der Hauptkomponentenanalyse ausgewertet. Es werden im wesentlichen die in der Spektralanalyse bekannten Methoden und zwar neben der sog. PCA-Methode (principal component analysis), d.h. der Hauptkomponentenanalyse, die sogenannte PLS-Methode (partial least square) oder MLR (multi linear regression), und darüber hinaus neuronale Netze verwendet. Speziell bei der Hauptkomponentenanalyse werden aus einer geeigneten Anzahl von Spektren, beispielsweise zwischen drei und zehn Spektren, sogenannte Scores zwecks Datenreduktion ausgewählt. Daraus werden die Kenngrößen PCl bis PCn ermittelt, die Eingangsgrößen insbesondere des Modells 30 gemäß Figur 3 sind.

Durch Einbindung der gemessenen Spektren in das Zustandsmodell oder in ein Prozeßmodell, die beispielsweise mit entsprechenden Maßzahlen den Zustand des Produktflusses oder aber auch die Qualität beschreiben, kann eine optimierte Prozeßführung geschaffen werden. Dabei finden die Produktzustände Eingang in die Prozeßmodellierung. Mit den Prozeßmodellen werden bevorzugt die am Ausgang des Prozesses zu erwartenden Produktzustände beschrieben. Dabei finden die am Eingang des Prozesses vorliegenden Produktzustände und die den Prozeßzustand beschreibenden Prozeßvariablen Verwendung.

Bei Realisierung der Bleiche als mehrstufiger Produktionsprozeß erweist es sich als vorteilhaft, auch ein mehrstufiges Modell einzusetzen.

Bei der Aufstellung der Modelle kann von neuronalen Netzen und/oder Fuzzy-Logik Gebrauch gemacht werden. Insbesondere geht es darum, die Gültigkeit der Modelle zu validieren, was durch ein online-Training der einzelnen Modelle bzw. der Teilmodelle erfolgen kann. Dabei kann es für die Praxis wichtig sein, durch rechnergestützte Auswahl aller informationstragender Daten eine Überprüfung der jeweils erhaltenen Ergebnisse vorzunehmen. Dieses Verfahren wurde als sog. "Novelty Detection" vorgeschlagen und ermöglicht, neue Datensätze in das Auswerteverfahren einzubringen. Bei Vorliegen nichtkonsistenter Ergebnisse ist ein Nachtrainieren der Modelle notwendig.

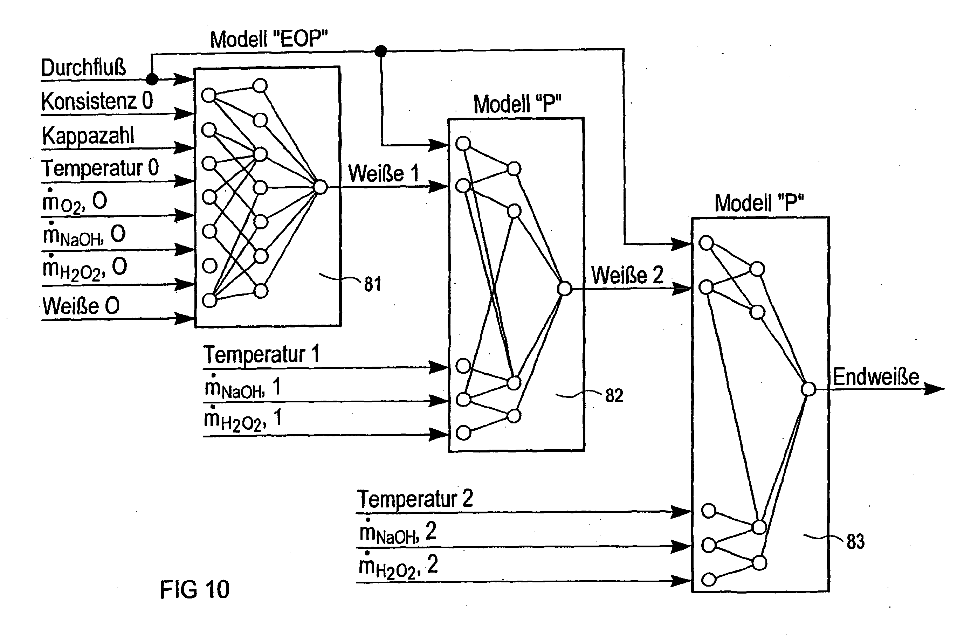

In Figur 10 ist dazu beispielhaft ein mehrstufiges statisches neuronales Netz mit den Stufen 81, 82 und 83 gezeigt. Dabei ist jeweils eine einzige Bleichstufe durch ein eigenes neuronales Netz beschrieben. Die Mehrstufigkeit der Bleichanlage wird dann durch das Zusammenschalten der einzelnen neuronalen Netze erreicht. Die Zustandsmaßzahl wird vorteilhaft als Kopplung zwischen den einzelnen Stufen benutzt. Beim Training des neuronalen Netzes können dabei die zwischen den Stufen gemessenen Qualitätszahlen benutzt werden. Es ist aber auch ein Training des gesamten Netzwerkes ohne eine Messung zwischen den Stufen möglich.

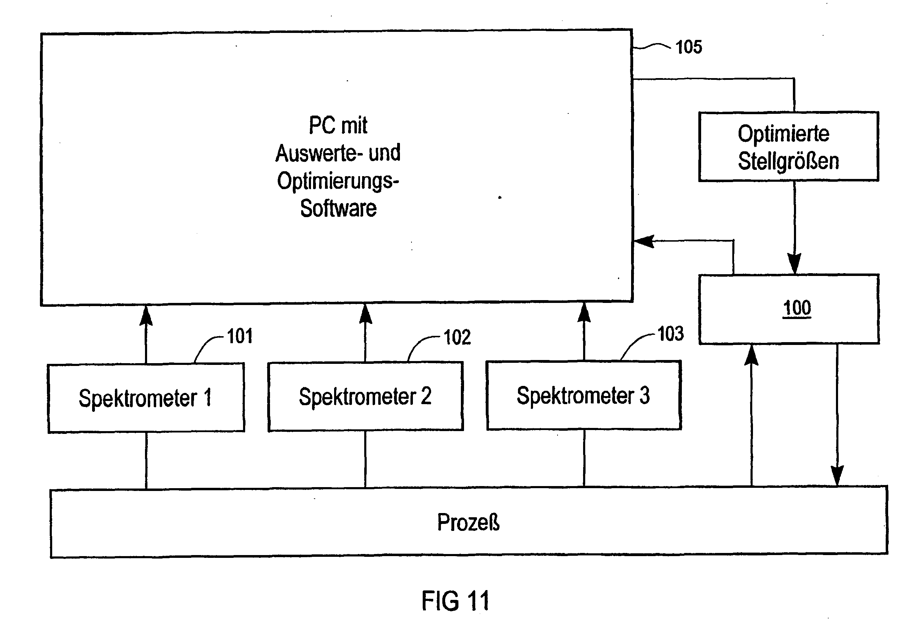

Figur 11 zeigt die Einbindung eines Rechners in eine Vorrichtung zur Optimierung der Prozeßführung beim Bleichen. Dabei kennzeichnet 101 bis 103 einzelne Spektrometer und 105 einen Rechner. Damit wird über entsprechende Auswerte- und Optimierungssoftware ein vorhandenes Prozeßleitsystem 100 beeinflußt. Es werden optimierte Stellgrößen erzeugt, die ein bekanntes Automatisierungsgerät als Prozeßleitsystem beaufschlagen, das in bekannter Weise mit der Bleichanlage zur Durchführung des Prozesses in Wechselwirkung steht. Die übliche Anlage wird also im wesentlichen durch die Spektrometer und das zugehörige Software-Paket, das auf üblichen Rechnern abläuft, ergänzt.

## Patentansprüche

1. Verfahren zur Prozeßführung und zur Prozeßoptimierung beim Bleichen von Faserstoffen, insbesondere Zellstoff, Holzstoff oder Altpapierstoff, durch Behandeln einer Suspension des Faserstoffes mit Bleichchemikalien unter Einsatz wenigstens eines Zustandsmodells und/oder Prozeßmodells, mit folgenden Merkmalen:
a) an mindestens einer Stelle werden am Faserstoff oder an der Stoffsuspension oder an einem aus der Stoffsuspension gewonnenen Probenblatt kontinuierliche Spektren von elektromagnetischer Strahlung und/oder kontinuierliche Spektren von mechanischen Eigenschaften gemessen,
b) durch mathematische Auswertung der kontinuierlichen Spektren werden Kenngrößen (PCl, ..., PCn) für die Stoffsuspension und/oder den Faserstoff gebildet,
c) die Kenngrößen (PCl, ..., PCn) und Labormessungen der dazugehörigen Produkteigenschaften werden in das zustandsmodell und/oder in das Prozeßmodell eingegeben, wobei bei Eingabe in das Prozeßmodell zusätzlich die Prozeßeigenschaften in das Prozeßmodell eingegeben werden, wodurch die Modelle verifiziert werden,
d) mit Hilfe des so gebildeten Zustands- und/oder Prozessmodells werden optimierte Stellgrößen für ein vorhandenes Prozessleitsystem (100) gebildet,
e) ein dynamisches Modell wird zur Überprüfung der durch ein statisches Modell optimierten Stellgrößen eingesetzt, wobei als eingesetztes dynamisches Modell ein neuronales Netz verwendet wird.

2. Verfahren nach Anspruch 1, **dadurch gekennzeichnet, daß** an mindestens einer Stelle am Faserstoff oder an der Stoffsuspension oder an einem aus der Stoffsuspension gewonnenen Probenblatt diskrete physikalische und/oder chemische Eigenschaften erfaßt werden.

3. Verfahren nach Anspruch 1 und Anspruch 2, **dadurch gekennzeichnet, daß** die diskreten physikalischen und/oder chemischen Eigenschaften zur Aurstellung des Zustandsmodells und gegebenenfalls des Prozeßmodells verwendet werden.

4. Verfahren nach Anspruch 1, **dadurch gekennzeichnet, daß** bei Wellenlängen der elektromagnetischen Strahlung zwischen 100 nm und 400 µm gemessen wird.

5. Verfahren nach Anspruch 4, **dadurch gekennzeichnet, daß** die elektromagnetische Strahlung als Absorptions-, Emissions-, Lumineszenz- oder als Raman-Spektrum erfaßt wird.

6. Verfahren nach Anspruch 4, **dadurch gekennzeichnet, daß** die elektromagnetische Strahlung in Transmission, direkter oder diffuser Reflexion oder gedämpfter Totalreflexion (ATR) erfaßt wird.

7. Verfahren nach Anspruch 1, **dadurch gekennzeichnet, daß** als kontinuierliche Spektren der mechanischen Eigenschaften die Faserlängenverteilung oder die Siebfraktionen der Fasern herangezogen werden.

8. Verfahren nach Anspruch 2, **dadurch gekennzeichnet, daß** als diskrete physikalische und/oder chemische Eigenschaften, die Faserstoffkonzentration bzw. deren Konsistenz, der Durchfluß und/oder die Temperatur der Stoffsuspension und ggfs. der Bleichchemikalien herangezogen werden.

9. Verfahren nach Anspruch 1, **dadurch gekennzeichnet, daß** an einer vorgegebenen Anzahl von Spektren eine Hauptkomponentenanalyse durchgeführt und zur Datenreduktion eine entsprechende Anzahl von Scores ausgewählt wird, und daß daraus die Kenngrößen für die Modellbildung ermittelt werden.

10. Verfahren nach Anspruch 9, **dadurch gekennzeichnet, daß** die Spektren vorverarbeitet und verdichtet werden, und daß die spezifischen Kennwerte der Spektren, insbesondere die Hauptkomponenten, zur Beschreibung des Produktzustandes ausgewählt und unmittelbar in das Zustandsmodell eingegeben werden.

11. Verfahren nach Anspruch 9, **dadurch gekennzeichnet, daß** die Spektren vorverarbeitet und verdichtet werden, daß die spezifischen Kennwerte der Spektren, insbesondere die Hauptkomponenten, in das Zustandsmodell eingebracht werden, und daß am Ausgang des Zustandsmodells die Produkteigenschaften gebildet und unmittelbar in das Prozeßmodell eingegeben werden.

12. Verfahren nach Anspruch 9, **dadurch gekennzeichnet, daß** für die Modellbildung ungeeignete Spektren durch Plausibilitätsprüfung eliminiert werden.

13. Verfahren nach Anspruch 1, **dadurch gekennzeichnet, daß** eine Bleiche aus mindestens einer Bleichstufe verwendet wird, wobei die Anzahl der hintereinander und/oder parallel geschalteten Bleichstufen nicht begrenzt ist.

14. Verfahren nach Anspruch 1, **dadurch gekennzeichnet, daß** die Messung der kontinuierlichen Spektren am Eingang in die Bleiche und/oder zwischen den einzelnen Bleichstufen und/oder danach erfolgt.

15. Verfahren nach Anspruch 1, **dadurch gekennzeichnet, daß** die durch Auswertung der Spektren erhaltenen Kenngrößen zur Steuerung und/oder Regelung der Bleichstufen herangezogen werden, wobei die Bleichstufen einzeln oder auch im Verbund optimiert werden.

16. Verfahren nach Anspruch 1, **dadurch gekennzeichnet, daß** zur Steuerung und/oder Regelung der Bleichstufen die Qualitätsparameter des gebleichten Stoffes, wie insbesondere die Weiße, Schmutzpunkte, Festigkeitseigenschaften wie Reißlänge, Kappazahl und/oder die Viskosität modelliert werden.

17. Verfahren nach Anspruch 1, **dadurch gekennzeichnet, daß** Neuronale Netze außer in Kombination mit spektrometrischen Messungen auch mit einer normalen Weißemessung eingesetzt werden.

18. Verfahren nach Anspruch 17, **dadurch gekennzeichnet, daß** die Modellansätze neben der Vorhersage der Produktqualität auch zur Berechnung der Chemikalieneinsätze herangezogen werden.

19. Verfahren nach Anspruch 18, **dadurch gekennzeichnet, daß** Teilmodelle entsprechend den eingesetzten Faserstoffen und den Sollwerten der Qualitätsparameter gebildet werden.

20. Verfahren nach Anspruch 1, wobei eine Steuerung und/oder Regelung der Bleichstufen erfolgt, **dadurch gekennzeichnet, daß** der Modellansatz mit den Qualitätsparametern in der Prozeßoptimierung eingesetzt wird.

21. Verfahren nach einem der vorhergehenden Ansprüche, **dadurch gekennzeichnet, daß** eine Kostenfunktion gebildet wird, die mit einem Optimierer durch geeignete Variation der Stellgrößen optimiert wird.

22. Verfahren nach einem der Ansprüche 20 oder 21, **dadurch gekennzeichnet, daß** die Optimierung durch genetische Algorithmen erfolgt.

23. Verfahren nach Anspruch 21, **dadurch gekennzeichnet, daß** als Kostenfunktion eine Kostenfunktion für die Produktionskosten und/oder eine Gewinnfunktion eingesetzt wird.

24. Verfahren nach einem der vorhergehenden Ansprüch, **dadurch gekennzeichnet, daß** zur Modellierung der Bleichsequenz ein mehrstufiges statisches neuronales Netz eingesetzt wird.

25. Verfahren nach Anspruch 24, **dadurch gekennzeichnet, daß** für jede Bleichstufe ein eigenes neuronales Netz definiert wird, wobei die neuronalen Netze über Produktzustandsmaßzahlen gekoppelt werden.

26. Verfahren nach einem der vorhergehenden Ansprüche, **dadurch gekennzeichnet, daß** das Modell und/oder die Teilmodelle online trainiert werden.

27. Verfahren nach einem der vorhergehenden Ansprüche, **dadurch gekennzeichnet, daß** durch rechnergestützte Auswahl aller informationstragenden Daten eine Überprüfung von erhaltenen Ergebnissen erfolgt("Novelty Detection").

28. Verfahren nach Anspruch 27, **dadurch gekennzeichnet, daß** bei Vorliegen nichtkonsistenter Ergebnisse ein Nachtrainieren erfolgt.

29. Vorrichtung zur Durchführung des Verfahrens nach einem der Änsprüche 1 bis 28, bestehend aus wenigstens einem Spektrometer (101, 102, 103) zur Messung von kontinuierlichen Spektren, aus einem Digitalrechner (105) zur mathematischen Auswertung der kontinuierlichen Spektren zwecks Ermittlung der Kenngrößen und zur Aufstellung des Zustandsmodells und/oder Prozeßmodells aus den Kenngrößen und gegebenenfalls den Prozeßeigenschaften, sowie aus einem Prozeßleitsystem (100) zur Prozeßoptimierung beim Bleichen von Faserstoffen unter Verwendung der optimierten Stellgrößen, wobei zur Überprüfung der durch ein statisches Modell optimierten Stellgrößen eine Einheit (79) mit einem als neuronales Netz ausgebildeten dynamischen Modell vorgesehen ist.

## Claims

1. Method for process management and for process optimisation in the bleaching of fibre pulps, in particular chemical pulp, wood pulp or recycled paper pulp, by treating a suspension of the fibre pulp with bleaching chemicals while using at least one state model and/or process model, said method having the following features:
a) continuous spectra of electromagnetic radiation and/or continuous spectra of mechanical properties are measured at at least one point on the fibre pulp or the pulp suspension or a sample sheet obtained from the pulp suspension,
b) characteristic quantities (PCl, ..., PCn) for the pulp suspension and/or the fibre pulp are formed by mathematical evaluation of the continuous spectra,
c) the characteristic quantities (PCl, ..., PCn) and laboratory measurements of the associated product properties are input into the state model and/or into the process model, wherein upon input into the process model the process properties are additionally input into the process model, by means of which the models are verified,
d) optimised manipulated variables for an existing process control system (100) are formed with the aid of the state and/or process model formed in this way,
e) a dynamic model is used for checking the manipulated variables optimised by means of a static model, with a neural network being used as the dynamic model.

2. Method according to claim 1, **characterised in that** discrete physical and/or chemical properties are recorded at at least one point on the fibre pulp or the pulp suspension or a sample sheet obtained from the pulp suspension.

3. Method according to claim 1 and claim 2, **characterised in that** the discrete physical and/or chemical properties are used for setting up the state model and optionally the process model.

4. Method according to claim 1, **characterised in that** measurements are taken at wavelengths of the electromagnetic radiation between 100 nm and 400 µm.

5. Method according to claim 4, **characterised in that** the electromagnetic radiation is recorded as an absorption, emission, luminescence or Raman spectrum.

6. Method according to claim 4, **characterised in that** the electromagnetic radiation is recorded in transmission, direct or diffuse reflection or attenuated total reflection (ATR).

7. Method according to claim 1, **characterised in that** the fibre length distribution or the screen fractions of the fibres are used as continuous spectra of the mechanical properties.

8. Method according to claim 2, **characterised in that** the fibre pulp concentration or its consistency, the flow rate and/or the temperature of the pulp suspension, and optionally of the bleaching chemicals, are used as discrete physical and/or chemical properties.

9. Method according to claim 1, **characterised in that** a principal component analysis is carried out on a predetermined number of spectra and, for data reduction purposes, an appropriate number of scores is selected, and **in that** the characteristic quantities for the model formation are determined therefrom.

10. Method according to claim 9, **characterised in that** the spectra are pre-processed and compressed, and **in that** the specific characteristic values of the spectra, in particular the principal components, are selected for describing the product state and are input directly into the state model.

11. Method according to claim 9, **characterised in that** the spectra are pre-processed and compressed, **in that** the specific characteristic values of the spectra, in particular the principal components, are incorporated into the state model, and **in that** the product properties are formed at the output of the state model and input directly into the process model.

12. Method according to claim 9, **characterised in that** spectra unsuitable for the model formation are eliminated by plausibility checking.

13. Method according to claim 1, **characterised in that** a bleach from at least one bleaching stage is used, with the number of bleaching stages that are connected in series and/or in parallel not being limited.

14. Method according to claim 1, **characterised in that** the continuous spectra are measured at the entry into the bleaching and/or between the individual bleaching stages and/or thereafter.

15. Method according to claim 1, **characterised in that** the characteristic quantities obtained by evaluating the spectra are used for feed forward and/or feedback control of the bleaching stages, with the bleaching stages being optimised individually or in combination.

16. Method according to claim 1, **characterised in that** the quality parameters of the bleached pulp, such as in particular the brightness, dirt specks, strength properties such as breaking length, kappa number and/or viscosity are modelled for the purpose of feed forward and/or feedback control of the bleaching stages.

17. Method according to claim 1, **characterised in that** neural networks are used not only in combination with spectrometric measurements but also with a normal brightness measurement.

18. Method according to claim 17, **characterised in that** the model approaches are used not only for predicting the product quality but also for calculating the chemical dosages.

19. Method according to claim 18, **characterised in that** sub-models are formed corresponding to the fibre pulps used and the setpoint values of the quality parameters.

20. Method according to claim 1, wherein feed forward and/or feedback control of the bleaching stages is carried out, **characterised in that** the model approach is used with the quality parameters in the process optimisation.

21. Method according to one of the preceding claims, **characterised in that** a cost function is formed and optimised by means of an optimiser through suitable variation of the manipulated variables.

22. Method according to one of claims 20 and 21, **characterised in that** the optimisation is carried out using genetic algorithms.

23. Method according to claim 21, **characterised in that** a cost function for the production costs and/or a profit function is used as the cost function.

24. Method according to one of the preceding claims, **characterised in that** a multistage static neural network is used for modelling the bleaching sequence.

25. Method according to claim 24, **characterised in that** a separate neural network is defined for each bleaching stage, with the neural networks being coupled via product state metrics.

26. Method according to one of the preceding claims, **characterised in that** the model and/or the sub-models are trained online.

27. Method according to one of the preceding claims, **characterised in that** a check of results obtained ("novelty detection") is carried out by computer-aided selection of all information-carrying data.

28. Method according to claim 27, **characterised in that** retraining is carried out when there are inconsistent results.

29. Device for carrying out the method according to one of claims 1 to 28, said device consisting of at least one spectrometer (101, 102, 103) for measuring continuous spectra, a digital computer (105) for mathematical evaluation of the continuous spectra aimed at determining the characteristic quantities and for setting up the state model and/or process model from the characteristic quantities and optionally the process properties, and a process control system (100) for process optimisation in the bleaching of fibre pulps while using the optimised manipulated variables, with a unit (79) having a dynamic model embodied as a neural network being provided for checking the manipulated variables optimised by means of a static model.

## Revendications

1. Procédé pour conduire un processus et pour optimiser un processus lors du blanchiment de matières fibreuses, notamment de la pâte de cellulose, de la pâte mécanique ou de la pâte de vieux papier, par traitement d'une suspension de la matière fibreuse par des produits chimiques de blanchiment en utilisant au moins un modèle d'état et/ou un modèle de processus ayant les caractéristiques suivantes :
a) on mesure en au moins, un point sur la matière fibreuse ou sur la suspension de matière ou sur une feuille d'échantillon obtenue à partir de la suspension de la matière des spectres continus de rayonnement électromagnétique et/ou des spectres continus de propriétés mécaniques,
b) on forme, en exploitant mathématiquement les spectres continus, des grandeurs caractéristiques (PCl, ..., PCn) pour la suspension de matière et/ou pour la matière fibreuse,
c) on entre les grandeurs caractéristiques (PCl, ..., PCn) et des mesures de laboratoire des propriétés associées au produit dans le modèle d'état et/ou dans le modèle de processus de manière à vérifier les modèles, dans lequel, lors de l'introduction dans le modèle de processus, on entre supplémentairement les propriétés de processus dans le modèle de processus,
d) à l'aide du modèle d'état et/ou de processus ainsi formé, on forme des grandeurs réglantes optimisées pour un système (100) existant de conduite de processus,
e) on utilise un modèle dynamique pour contrôler les grandeurs réglantes optimisées par un modèle statique en utilisant un réseau neuronal comme modèle dynamique mis en oeuvre.

2. Procédé suivant la revendication 1, **caractérisé en ce que** l'on détecte, en au moins un point sur la matière fibreuse ou sur la suspension de matière ou sur une feuille échantillon obtenue à partir de la suspension de matière, des propriétés physiques et/ou des propriétés chimiques discrètes.

3. Procédé suivant les revendications 1 et 2, **caractérisé en ce que** l'on utilise les propriétés physiques et/ou chimiques discrètes pour établir le modèle d'état et, le cas échéant, le modèle de processus.

4. Procédé suivant la revendication 1, **caractérisé en ce que** l'on mesure à des longueurs d'onde du rayonnement électromagnétique comprises entre 100 nm et 400 µm.

5. Procédé suivant la revendication 4, **caractérisé en ce que** l'on détecte le rayonnement électromagnétique en tant que spectre d'absorption, d'émission, de luminescence ou de spectre Raman.

6. Procédé suivant la revendication 4, **caractérisé en ce que** l'on détecte le rayonnement électromagnétique en transmission, en réflexion directe ou diffuse ou en réflexion totale atténuée (ATR).

7. Procédé suivant la revendication 1, **caractérisé en ce que** l'on utilise, comme spectres continus des propriétés mécaniques, la répartition de longueur des fibres ou les fractions granulométriques des fibres.

8. Procédé suivant la revendication 2, **caractérisé en ce que** l'on utilise, comme propriétés physiques et/ou chimiques discrètes, la concentration en matière fibreuse ou sa consistance, le débit et/ou la température de la suspension de matière et, le cas échéant, les produits chimiques de blanchiment.

9. Procédé suivant la revendication 1, **caractérisé en ce que** l'on effectue, sur un nombre donné à l'avance de spectres, une analyse de composantes principales et, pour la réduction des données, on choisit un nombre adéquat de scores et on en détermine les grandeurs caractéristiques pour la formation du modèle.

10. Procédé suivant la revendication 9, **caractérisé en ce que** l'on prétraite les spectres et on les comprime et **en ce que** l'on sélectionne les valeurs caractéristiques spécifiques des spectres, notamment les composantes principales pour décrire l'état du produit et on les entre directement dans le modèle d'état.

11. Procédé suivant la revendication 9, **caractérisé en ce que** l'on prétraite et comprime les spectres, **en ce que** l'on introduit les valeurs caractéristiques spécifiques des spectres, notamment les composantes principales, dans le modèle d'état, et **en ce que** l'on forme à la sortie du modèle d'état les propriétés du produit et on les introduit directement dans le modèle de processus.

12. Procédé suivant la revendication 9, **caractérisé en ce que**, pour la formation du modèle, on élimine les spectres qui ne conviennent pas par vérification de plausibilité.

13. Procédé suivant la revendication 1, **caractérisé en ce que** l'on utilise un blanchiment constitué d'au moins un étage de blanchiment, le nombre des étages de blanchiment montés l'un derrière l'autre et/ou en parallèle n'étant pas limité.

14. Procédé suivant la revendication 1, **caractérisé en ce que** l'on effectue la mesure des spectres continus à l'entrée dans le blanchiment et/ou entre les étages individuels de blanchiment et/ou après.

15. Procédé suivant la revendication 1, **caractérisé en ce que** l'on utilise les grandeurs caractéristiques obtenues par l'exploitation des spectres pour commander et/ou pour réguler les étages de blanchiment, les étages de blanchiment étant optimisés individuellement ou également en combinaison.

16. Procédé suivant la revendication 1, **caractérisé en ce que**, pour commander et/ou réguler les étages de blanchiment, on modélise les paramètres de qualité de la matière blanche comme notamment les blancheurs, les points d'impuretés, les propriétés de résistance mécanique comme la longueur de rupture, l'indice kappa et/ou la viscosité.

17. Procédé suivant la revendication 1, **caractérisé en ce que** l'on utilise des réseaux neuronaux, outre en combinaison avec des mesures spectrométriques, également avec une mesure normale de blancheur.

18. Procédé suivant la revendication 17, **caractérisé en ce que** l'on utilise les équations du modèle, outre pour prédire la qualité du produit, également pour calculer les apports de produits chimiques.

19. Procédé suivant la revendication 18, **caractérisé en ce que** l'on forme des sous-modèles en fonction des matières fibreuses utilisées et des valeurs de consigne des paramètres de qualité.

20. Procédé suivant la revendication 1, dans lequel on effectue une commande et/ou une régulation des étages de blanchiment, **caractérisé en ce que** l'on utilise l'équation du modèle ayant les paramètres de qualité dans l'optimisation du procédé.

21. Procédé suivant l'une des revendications précédentes, **caractérisé en ce que** l'on forme une fonction de coût que l'on optimise avec un optimiseur en faisant varier d'une manière appropriée les grandeurs réglantes.

22. Procédé suivant l'une des revendications 20 ou 21, **caractérisé en ce que** l'on effectue l'optimisation par des algorithmes génétiques.

23. Procédé suivant la revendication 21, **caractérisé en ce que** l'on utilise, comme fonction de coût, une fonction de coût pour les coûts de production et/ou une fonction de profit.

24. Procédé suivant l'une des revendications précédentes, **caractérisé en ce que** l'on met en oeuvre, pour la modélisation de la séquence de blanchiment, un réseau neuronal statique à plusieurs étages.

25. Procédé suivant la revendication 24, **caractérisé en ce que** l'on définit, pour chaque étage de blanchiment, son propre réseau neuronal, les réseaux neuronaux étant couplés par des indices d'états produits.

26. Procédé suivant l'une des revendications précédentes, **caractérisé en ce que** l'on fait subir,au modèle et/ou aux sous-modèles, un apprentissage on-line.

27. Procédé suivant l'une des revendications précédentes, **caractérisé en ce qu'**il s'effectue, par une sélection assistée par ordinateur de toutes les données porteuses d'informations, un contrôle des résultats obtenus ("Novelty Détection").

28. Procédé suivant la revendication 27, **caractérisé en ce qu'**il s'effectue un post-apprentissage en présence de résultats non consistants.

29. Dispositif pour la mise en oeuvre du procédé suivant l'une des revendications 1 à 28, constitué d'au moins un spectromètre (101,102,103) de mesure de spectres continus, d'un ordinateur (105) numérique d'exploitation mathématique des spectres continus en vue de déterminer les grandeurs caractéristiques et d'établir le modèle d'état et/ou le modèle de processus à partir des grandeurs caractéristiques et, le cas échéant, des propriétés de processus ainsi que d'un système (100) de conduite de processus destiné à optimiser le processus lors du blanchiment de matières fibreuses en utilisant les grandeurs réglantes optimisées, dans lequel, pour contrôler les grandeurs réglantes optimisées par un modèle statique, il est prévu une unité (79) ayant un modèle dynamique constitué sous la forme d'un réseau neuronal.
